# EUROPEAN PATENT SPECIFICATION

(11) **EP 0 997 734 B1**
(45) Date of publication and mention of the grant of the patent: **27.09.2006**
(21) Application number: 98890317.5
(22) Date of filing: 29.10.1998
(51) Int. Cl.: G01N 33/569

(54) **Composition useful for the early diagnosis of visceral leishmaniasis and a process for preparing the same**
Zusammensetzung zur Früherkennung von viszeraler Leishmaniose und Verfahren zu seiner Herstellung
Composition utilisable pour le diagnostic précoce de la Leishmaniose viscérale et le procédé de sa préparation

(43) Date of publication of application: 03.05.2000
(73) Proprietor: COUNCIL OF SCIENTIFIC AND INDUSTRIAL RESEARCH, New Delhi 110 001 (IN)
(72) Inventor: Girish, Kumar Jain, Lucknow 226 001 (IN); Suman, Tiwari, Lucknow 226 001 (IN); Suman, Gupta, Lucknow 226 001 (IN); Katiyar, Jagdish Chandra, Locknow 226 001 (IN)
(74) Representative: Schwarz, Albin

(56) References cited:
- WO-A-93/00807
- WO-A-95/11700
- MENGISTU G., KIESSLING R., AKUFFO H. : "The value of a direct agglutination test in the diagnosis of cutaneous and visceral leishmaniasis in Ethiopia" TRANSACTIONS OF THE ROYAL SOCIETY OF TROPICAL MEDICINE AND HYGIENE, vol. 84, 1990, pages 359-362, XP002101645
- DATABASE CHEMABS CHEMICAL ABSTRACTS SERVICE, COLUMBUS, OHIO, US PALATNIK DE SOUZA, CLARISA B. ET AL: "Preparation containing the Leishmania cell fraction antigen FML (fucose-mannose ligand) and the use of the FML antigen and its subfractions and components for diagnosis of visceral leishmaniasis of humans and animals, and for vaccination, treatment, or immunotherapy of visceral leishmaniasis in huma" XP002101647
- PALATNIK-DE-SOUZA CLARISA B. ET AL: "The FML vaccine (fucose-mannose ligand) protects hamsters from experimental kala-azar" JOURNAL OF THE BRAZILIAN ASSOCIATION FOR THE ADVANCEMNET OF SCIENCE, vol. 46, no. 4, 1994, pages 290-296, XP002101646 BR
- MANNING M C ET AL: "APPROACHES FOR INCREASING THE SOLUTION STABILITY OF PROTEINS" BIOTECHNOLOGY AND BIOENGINEERING, vol. 48, no. 5, 5 December 1995, pages 506-512, XP000651762

## Description

### Field of the invention

This invention relates to a composition containing Leishmania promastigotes and a protein stabilizing solute useful for the early diagnosis of visceral leishmaniasis. Particularly, this invention relates to a composition containing *Leishmania donovani* promastigotes and a protein stabilising solute useful for the early diagnosis of visceral leishmaniasis or kala-azar. More particularly, the invention is useful for the early diagnosis of visceral leishmaniasis or Kala-azar in field conditions using Direct Agglutination Test (DAT) based on the composition of as disclosed herein.

### Background of the invention

Visceral Leishmaniasis or Kala-azar, caused by an intracellular protozoan parasite Leishmania donovani, is transmitted from man to man through *Phlebotomus argentipes* (sand fly) of the phlebotomidae family.

The disease is geographically distributed in tropics and substropics of the world extending through most of the central and South East Asia, India, China, the Mediterranean region and Africa 90% of all the cases occur in Bangladesh, Brazil, India and sudan.

Visceral leishmaniasis is caused by *Leishmania donovani* in the Indian subcontinent and in East Africa, by *Leishmania infantum* in the mediterranean region and by *Leishmania chagasi* in the New World, mainly in Brazil, Peru and Paraguay (J. D. Berman; Clin. Infect. Dis., 24, 684, 1997).

It has been estimated that about 15 million people carry Leishmania infection (R.W. Ashford, P.Deszeux, P. deRadt; Parasitol. Today, 8, 104, 1992) and over 400,000 new cases appear each year. The number of people at risk is estimated to be 350 million in about 88 countires (Anon.; Tropical Disease, Leishmaniasis, 14, 1991 and F. Modabber; Tropical Disease Research Programme, Eleventh Programme Report, page 77, 1992). In India, in 1991 the disease had posed a great threat involving 38 out of the 42 districts of Bihar state, 8 districts of West Bengal and 2 districts of Eastern Uttar Pradesh (TDR News Letter, Published by UNDP/World Bank/WHO Special Programme for Research and Training in Tropical Diseases No. 36, 1991 and V. Srivastava; Ph.D. Thesis; Chemotherapy and Immunodiagnostic Investigations on Visceral Leishmaniasis, Agra University, Agra, India, 1992). From Bihar alone in 1991 the number of cases recorded were 2,50,000 with 2 to 3 thousand deaths and more than 4.5 lakh are directly exposed to the parasite (C.P. Thakur; Status Paper on Kala-azar in Bihar, Core group discussion on Kala-azar control in India, 24-25 September 1993). Sporadic cases have also been reported from Gujrat (F.B.W. Gajwani, A. Mehta, B.A. Sayed, N.N. Patel and R.S. Pandey; Ind. Med. Assoc., 49, 216, 1967), Kashmir (V.P. Jacob and S.L. Kalra; Ind. J. Med. Res., 39, 329, 1951), Himachal Pradesh (S. Gupta and R.S. Bhatia; Ind. Practitioner, 28, 609, 1975), Delhi (S.R. Naik, P.M. Rao, D.V. Dutta, S. Mehta, and P.N. Chuttani; Trans. R. Soc. Trop. Med. Hyg., 33, 61, 1979) and Uttar Pradesh (V. Srivastava; Ph.D. Thesis; Chemotherapy and Immunodiagnostic Investigations on Visceral Leishmaniasis, Agra University, Agra, India, 1992 and S. Gupta, S. Tiwari, A.K. Bagchi and J. C. Katiyar; Current Science 73,456, 1997).

The disease is characterized by irregular bouts of fever, often with loss of weight and appetite, hepatosplenomegaly, leucopenia, cachexia etc. If left untreated the fatality rate can be as high as 100%. In early stages the disease can be effectively treated with proper course of sodium stibogluconate (SSG) as there is little or no morbidity and no immunosuppression. At later stages disease is assoicated with many problems like greater morbidity, immunosuppression, secondary infection and drug resistance etc. and at this stage the patients respond poorly to various combinations of chemotherapy.

Keeping in view of these limitations, the essential component in the management of the disease is the early detection and proper treatment with available chemotherapeutic agents.

### Prior art references

Till date, there is no suitable method for the diagnosis of Kala-azar particularly in early stages.

The definitive diagnosis of visceral leishmaniasis is based on demonstration of parasites in biopsies or aspirates of infected spleen, bone marrow and lymph nodes. Culture of aspirates from these locations is also sometimes successful. Both approaches suffer because of lack of adequate sensitivity when the load of organism is low. Spleen biopsy detects 90% active cases whereas bone marrow aspirate can detect 60-70% active cases. By lymph gland biopsy only 50-60% active cases can be detected. These procedures at times are hazardous, painful and labour extensive, require skilled hands, possible in hospitals only where results could be available at least 24 hours later, costly, fail to detect early and slight infective stage.

For Kala-azar, some of the non-specific tests which were in use in early days and being practiced even today at places include; Formal gel or Aldehyde test and Chopra's antimony test. They are also related to the level of IgG and positive results are also obtained when other causes of raised immunoglobulin levels are present whether of IgG or IgM classes. It is therefore a screening test, which, if positive, requires further investigations. The test is positive in about 85% of patients with VL. The test becomes positive about three months after the infection. So these tests may detect only symptomatic cases, they fail to detect early leishmaniasis.

Serological tests are a useful adjunct and are specially valuable in early or highly immune cases when amastigotes may be present in insufficient numbers to be seen easly. Besides, these tests have the advantages that the blood sampling is relatively easy and can be performed with little inconvenience to the patient and many samples may be processed simultaneously.

A battery of immunological procedures have been developed and they demonstrate antileishmanial antibodies for confirming clinical diagnosis. These include Indirect Immunofluorescent Antibody Test (C.L.Jaffe and D. McMahon Pratt.; Trans. R. Soc. Trop. Med. Hyg., 81, 587, 1987), Enzyme Linked Immunosborent Assay (A. Jahn and H.J. Diesfeld; Trans. R. Soc. Trop. Med. Hyg., 77, 451, 1983 and E.R.M. El Amin, E.P. Wright, P.A. Kager, J.J. Laarman and K.W. Pondman; Trans. R. Soc. Trop. Med. Hyg., 79, 344, 1985), Counter Current Immunoelectrophoresis (J. Kohanteb, S.M. Ardehali and M. R. Rijai; Trans. R. Soc. Trap. Med. Hyg., 81, 578, 1987) and Complement Fixation Test (M.G. Pappas, L.T. Canon, W.T. Hakmeyer and D.H. Smith; Ann. Trop. Med. Parasitol., 79, 147, 1985). However, some have low sensitivity, others are cross reactive and still others require elaborate laboratory facilities (IFA and RIA). Thus they do not meet the requirements of field test as mentioned below.

An ideal immunodiagnostic test should be simple, quick, specific and cheap, and be applicable in the fields even in the adverse conditions that prevail in many areas where leishmaniasis is endemic.

Direct Agglutination Test (A.E. Harith, A.H.Z. Kolk, P.A. Kager, J. Leeuwenburg, R. Muigai, S. Kingu and J.J. Laarman; Trans. R. Soc. Trop. Med. Hyg.; 80, 583, 1986 and A.E. Harith, A.H.Z. Kolk, J. Leeuwenburg, R. Muigai, E. Huigen, T. Jelsma and P.A. Kager; J. Clin. Microbiol.; 26, 1321, 1988) is promising in meeting these requirements. In DAT the antigen preparation consists of whole organism and the serological response to surface borne antigen is measured and the test could be performed on samples of whole blood, thus the difficulties of preparation and storage of serum, plasma and blood in filter paper are avoided. Allain and Kagan first described DAT (S.Allan and I.G. Kagan; Trop. Med. Hyg., 24, 332, 1975) for diagnosis of visceral leishmaniasis,

Since 1986, DAT was modified and simplified to increase sensitivity and specificity and also comparisons were made with other serological tests. The test is also capable of detecting canine visceral leishmaniasis (A.E. Harith, R.J. Slappendel, I. Reiter, F. van Knappen, P. de Korte, E. Huigen and A.H.J. Kolk; J. Clin. Microbiol., 27, 2252, 1989) and diffused cutaneous leishmaniasis (G. Mengistu, R. Kisseling and H. Akuffo; Trans. R. Soc. Trop. Med. Hyg., 84, 359, 1990).

The studies have also demonstrated that while direct agglutination test (DAT) and enzyme linked immunosorbent assay (ELISA) have comparable diagnostic potentials, the former being more specific (S. Gupta, J.K. Srivastava, A.Pal, J.C. Katiyar, K.C. Saxena and B.N.Dhawan; Serodiag. Immunother. Infect. Dis., 6, 54, 1994) Besides, it neither requires sophisticated equipments/reagents nor high technical skill. The easy performance and the cost-effectivity adds to the merits of DAT for its acceptability for field use under Indian conditions.

The Direct Agglutination Test makes use of an aqueous suspension of trypsinized, Coomassie Brilliant Blue stained and formalized Leishmania donovani promastigotes, also referred as AQ Antigen or Liquid Antigen, for early diagnosis of visceral leishmaniasis. Preparation of aqueous antigen from other Leishmania species has also been reported in literature with a view to assess their comparable reactivity. Literature information reveals that there was no difference in titres obtained with antigen prepared from different Leishmania species (G. Mengistu, R. Kisseling and H. Akuffo; Trans. R. Soc. Trop. Med. Hyg., 84, 359, 1990). DAT is a fast and simple technique with a high sensitivity and specificity and, therefore, it is very suitable for use in field. One of the major drawback of the Liquid Antigen based DAT is its limited stability. Secondly, it suffers from the disadvantage that it has to be stored at 4° C, and a cold chain is required for its stability. The results are not repeated if the cold chain is disrupted at any point. The shaking during transportation also significantly affects the reproducibility of results with aqueous antigen. Therefore, when no cooling facilities are available and excessive road transportation is involved, as is often the case in the areas where leishmaniasis is frequently encountered, the availability of stable solid form of the antigen would facilitate the use of Direct Agglutination Test for the early diagnosis of visceral leishmaniasis in field conditions.

It was observed that the aqueous antigen on drying under high vaccum at room temperature produces a solid powder which does not show agglutination with Leishmania donovani antibodies and is, therefore, not useful in the early diagnosis of visceral leishmaniasis by direct agglutination test.

Similarly freezing the aqueous antigen at temperatures ranging from 0° C to -196° C and subsequently freeze drying the frozen antigen also results into a solid cake which also does not show any reactivity with Leishmania donovani antibodies and thus can not find use in early diagnosis of visceral leishmaniasis by direct agglutination test.

Thus, under the above mentioned conditions the aqueous antigen is undergoing denaturation rendering it inactive for early diagnosis of visceral leishamaniasis.

In the former case the denaturation of antigen may be taking place due to mechanical stresses produced under high vacuum in liquid state which may be producing non native conformation in dried state, whereas in the later case the major cause of loss in reactivity may be due to surface and freeze induced denaturation of protein present in Leishmania donovani antigen during freezing and freeze drying which again may be due to the formation of non native conformation in dry state.

Freeze induced denaturation of protein and antigen is a major concern in their development as pharmaceuticals and diagnostic tools. Freezing plays a crucial role in the damage incurred by the protein and antigens during freeze drying. The freeze induced denaturation of proteins and antigens is very closely related to surface induced denaturation and takes place mainly during the freezing and freeze drying process due to the stresses produced during these processes.

Freezing and freeze drying induced stresses, particularly cold temperatures, generation of large ice-water interface, exposure to concentrated solutes due to crystallization of water, crystallization of solutes and the resulting change in pH, are the main cause of denaturation of proteins and antigens which ultimately reduces the stability of the native state of proteins and antigens.

Since the preparation of Leishmania donovani antigen involves the use of whole organism and the serological response to surface borne antigen is measured in Direct Agglutination Test, it is highly important to protect the surface borne proteins from denaturation during freezing and subsequent freze drying process.

The freeze induced or surface induced denaturation of proteins and antigens can be inhibited by including protein stabilizing solutes in the compositions and formulations and the resistance to the stresses can be increased if the solution conditions are chosen that increase the thermodynamic stability of the native state of the proteins and antigens during freezing, freeze drying, storage and rehydration.

The requisite increase in free energy of denaturation during freezing and freeze drying can be achieved by adding requisite amount of protein stabilizing solutes to the proteins or antigens before initiating freezing or freeze drying. These solutes preferentially remain excluded from the surface of proteins at 25° C but the stabilizing effect of these solutes is maintained during freezing and freeze drying and free energy of proteins and antigens is increased which increases the stability of the native state.

The protein stabilising solutes mainly include surface active agents, sugars, polyols, polymers, aminoacids and salts. The surface active agents have been broadly grouped as anionic, cationic and nonionic surface active agents. There are reports of stabilization of enzyme substrates and protein molecules by polyanion additives ( M.C. Manning, J.E. Matsuura, B.S. Kendrick, J.D.Meyer, J.J. Dormish, M. Vrkljan, J.R. Ruth, J.F. Carpenter and E. Shefter; Biotechnology and Bioengineering, 48, 506, 1995) but stabilization of Leishmania donovani antigen using any of such additive materials is hitherto not reported in literature.

During the course of the Applicants studies, it was observed that the surface and freeze induced denaturation of Leishmania donovani antigen is inhibited by the addition of small amounts of protein stabilising solutes, particularly anionic surfactants, more particularly sodium lauryl sulphate, which is hitherto not known in the literature. The addition of sodium lauryl sulphate presumably serves as water substitute and hydrogen bond to Leishmania donovani antigen thereby increasing its free energy during freezing thus increasing the stabiliy of the native antigen which is subsequently maintained during the process of freeze drying resulting in a solid freeze dried cake which is not only stable at room temperature but also shows similar order of reactivity with Leishmania donovani antibodies as is observed with aqueous antigen. This clearly indicates that freezing and subsequent freeze drying in presence of sodium lauryl sulphate results in preservation of native structure of the Leishmania donovani antigen through four distinct stages namely freezing, freeze drying, storage and rehydration. The solid cake composition thus produced is useful for early diagnosis of visceral Leishmaniasis under field conditions in endemic areas.

The comparison of Infra Red spectrum of Leishmania donovani aqueous antigen, Leishmania donovani antigen freeze dried without sodium layryl sulphate and Leishmania donovani antigen freeze dried with sodium lauryl sulphate revealed that the antigen freeze dried in presence of sodium lauryl sulphate maintains the native conformation of Leishmania donovani antigen during freezing, freeze drying and rehydration. It is quite evident from the amide-I region of the IR spectrum which particularly shows bands corresponding mainly to C=O stretching vibrations and to a lesser extent to C=N stretchings and C-C-N bending vibrations. This region is sensitive to small vibrations in molecular geometry, secondary structural compositions and hydrogen bonding pattern within the proteins and antigenic substrates.

The IR spectrum of *Leishmania donovani* antigen freeze dried with sodium lauryl sulphate showed strong band at 1598 cm⁻¹ and a shoulder at 1675 cm⁻¹ where as IR spectrum of *Leishmania donovani* antigen without sodium lauryl sulphate showed appearence of an additional shoulder at 1625 cm⁻¹, decrease in intensity of band at 1675 cm⁻¹ and shifting of band at 1598 cm⁻¹ to 1594 cm⁻¹. The appearence of an additional shoulder at 1625 cm⁻¹ in *Leishmania donovani* antigen freeze dried without sodium lauryl sulphate may be assigned to alterations in secondary structures particularly to intermolecular B-sheet structure. Thus, the addition of sodium lauryl sulphate to *Leishmania donovani* antigen before freezing and freeze drying shows distinct protective effect in preserving the native conformation of the antigen.

### Objects of the invention

In order to overcome the problems associated with Aqueous Antigen or Liquid Antigen prepared from Leishmania promastigotes, the present invention provides a composition containing *Leishmania donovani* Promastigotes, *Leishmania infantum* Promastigotes and *Leishmania chagasi* Promastigotes, and a protein stabilising solute to stabilize *Leishmania donovani* promastigotes.

The main object of the present invention is to provide the *Leishmania donovani, Leishmania infantum and Leishmania chagasi* antigen in stable solid form in order to enhance its application potential even in unfavourable field conditions more particularly in endemic areas.

Another object of the present invention is to provide a freeze-dried composition containing *Leishmania donovani* promastigotes and a protein stabilising solute for the early diagnosis of visceral leishmaniasis which obviates the drawbacks as detailed above.

One more object of the invention is to provide the process for the preparing the above freeze-dried composition by freezing the solution at the temperature between 0 to-196 °C, freeze drying the frozen solution at a temperature between 0 to -100°C and storing freeze dried or lyophilized composition at a temperature between 18 to 45° C.

Still another object of the invention is to provide a solid, freeze-dried composition containing Leishmaia donovani promastigotes and a protein stabilising solute which is in solid form.

Yet another object of the invention is to provide a solid, freeze-dried composition containing Leishmania donovani promastigotes and a protein stabilising solute which is stable at room temperature.

Further object of this invention is to provide a solid, freeze-dried composition containing Leishmania donovani promasigotes and a protein stabilising solute which is specific for diagnosis of Leishmania donovani infection by Direct Agglutination Test.

Still further object of this invention is to provide a solid composition obtained by a freeze drying process and containing Leishmania donovani promastigotes and a protein stabilising solute which does not show cross reactivity with antibodies present in serum samples of the patients from other diseases such as: Tuberculosis, Leprosy, Amoebiasis; Giardiasis, Malaria, Trypanosomiasis and Filariasis and other parasitic diseases.

Yet further object of this invention is to provide a solid, freeze-dried composition containing Leismania donovani promastigotes and a protein stabilising solute which will serve as a convenient, rapid, simple economically feasible, sensitive and specific procedure for early diagnosis of Leishmania donovani infection in human cases under field conditions.

### Detailed Description of the invention

Accordingly the present invention provides a composition useful for the early diagnosis of visceral leishmaniasis as set out in claim 1.

The present invention also provides a process for the preparation of a solid, freeze-dried according to the invention composition which comprises : preparing aqueous antigen by methods known per se and treating the said aqueous antigen with che solution of protein stabilising solute in buffer, freezing the resulting solution, freeze drying or lyophilising the frozen solution and storing the brilliant blue coloured freeze dried or lyophilized composition in sterile glass containers or glass injection vials at ambient temperatures.

In an embodiment of the invention Leishmania promastigotes used for the preparation of antigen may be selected from Leishmania donovani, Leishmania infantum and Leishmania chagasi.

In another embodiment of the invention each one ml of aqueous antigen may contain Leishmania promastigotes in the range of 0.5 million to 100 million.

In yet another embodiment of the invention the composition may contain Leishmania promastigotes and the protein stabilising solute in the ratios ranging from of 5 million : 0.0001 mg to 100 million : 1.00 mg.

In another embodiment of the invention the buffers used may be such as physiological saline or citrate saline.

In another embodiment of the invention the freeze drying temperature of the frozen solution containing Leishmania donovani aqueous antigen and protein stabilising solute may be in the range of 0 °C to -100 °C.

According to the invention the protein stabilizing solute used is selected from sodium lauryl sulphate, sodium dodecyl sulphate, and sucrose.

To use the Composition of the invention, the composition equivalent to 0.5 million to 100 million promastigotes is taken in 10 ul to 100 ul of citrate saline buffer containing 0.1 ul to 100 ul of 0.01% to 1% formalin solution in citrate saline and added to 10 ul to 100 ul of test serum sample, placed in V-shaped well microtitre plates. The contents are mixed well and kept at 18° C to 22° C for 2-20 hrs and the results of agglutination are read visually against a white back ground. A titre of 1:3200 or more was considered positive. The results are compared with control pool positive and pool negative sera. The composition did not show any cross reactivity with sera from patients with other diseases like tuberculosis, leprosy, amoebiasis, giardiasis, malaria, filariasis and echinococcosis, hepatitis-B, typhoid, AIDS and trypanosomiasis. The dignostic tests can be carried out by those with ordinary skill in the art.

Aqueous Antigen or Liquid antigen is prepared by culturing spleen tissue (A.E. Harith, A.H.J. Kolk, P.A. Kager, J. Leeuwenburg, R. Muigai, S. Kiugu, S. Kiugu and J.J. Laarman; Trans. R. Soc. Trop. Med. Hyg., 80, 583, 1986) or bone marrow aspirate (S. Bhatnagar, S. C. Moitra, P.Y. Guru and J.C. Katiyar; Ind. J. Med. Res., 89, 439, 1989) from infected hamster. In brief, in-vitro, Leishmania donovani promastigotes were primarily grown by culturing infected hamster's spleen tissue in biphasic NNN medium at 26°C ± 1°C for 10 to 15 days. At every 7-8 days, Serial sub cultures of promastigotes are done by inoculating the biphasic NNN medium, consisting of 2 ml of NNN Medium and 2 ml of RPMI 1640 overlay medium with 1x10⁶ to 1.5x10⁶ million promastigotes. For bulk cultivation the promastigotes from NNN/RPMI 1640 medium are grown in L-15 monophasic medium containing 10% Foetal Calf Serum. Parasites are harvested when a vast majority of promastigote population attains elongated form and their concentration reaches about 40x10⁶ promastigote per ml of the medium. The culture is centrifuged for 15 minutes at 4000 g at 4° C. The pellet formed after centrifugation is washed five times by centrifugation with 200 equal volumes of cold Locke's solution at 3200 g at 4° C for 10 minutes. One packed volume of promastigotes is treated with 20 equal volumes of 0.2% trypsin in Locke's solution (pH 7.2) for 45 minutes at 37° C and then centrifuged at 3200 g at 4° C and washed five times as above. The pellet is then suspended in cold Locke's solution so as to obtain a concentration of 200 x 10⁶ promastigotes per ml. An equal volume of cold 2% formaldehyde in Locke's solution is added and left at 4° C for 20 hours for fixation. The promastigotes are then centrifuged at 3200 g and washed with cold citrate saline. These promastigotes are stained with 0.1% (w/v) coomassie brilliant blue in citrate saline for 90 minutes using a magnetic stirrer at a moderate speed. The stained parasites are then centrifuged and washed twice with citrate saline and the pellet is resuspended in citrate saline in such a way so as to obtain the Aqueous Antigen Suspension having 5 million to 100 million promastigotes per ml.

The details of the invention are given in the example provided below which are given by way of illustrations only and therefore, should not be construed to limit the scope of the present invention.

### Example 1:

### Preparation of AQ Antigen or Liquid Antigen

In-vitro, Leishmania donovani promastigotes were primarily grown by culturing infected hamster's spleen tissue in biphasic NNN medium at 26 °C ± 1 °C for 10 to 15 days. At every 7-8 days, Serial sub cultures of promastigotes are done by inoculating 4 ml of biphasic NNN medium, consisting of 2ml of NNN Medium and 2 ml of RPMI 1640 Overlay Medium with 1x10⁶ to 1.5x10⁶ million promastigotes. Bulk cultivation of the promastigotes is done by inoculating 3 ml of NNN/RPMI 1640 sub culture containing 5x10⁶ promastigotes per ml in 50 ml of L-15 monophasic medium containing 10% Foetal Calf Serum. Parasites are harvested when a vast majority of promastigote population attains elongated form and their concentration reaches about 40x10⁶ promastigote per ml of the medium. The culture is centrifuged for 15 minutes at 4000 g at 4° C. The pellet formed after centrifugation is washed five times by centrifugation with 200 equal volumes of cold Locke's solution at 3200 g at 4° C for 10 minutes. One packed volume of promastigotes is treated with 20 equal volumes of 0.2% trypsin in Locke's solution (pH 7.2) for 45 minutes at 37 °C and then centrifuged at 3200 g at 4° C and washed five times as above. The pellet is then suspended in cold Locke's solution so as to obtain a concentration of 200 x 10⁶ promastigotes per ml. An equal volume of cold 2% formaldehyde in Locke's solution is added and left at 4° C for 20 hours for fixation. The promastigotes are then centrifuged at 3200 g and washed with cold citrate saline. These promastigotes are stained with 0.1% (w/v) coomassie brilliant blue in citrate saline for 90 minutes using a magnetic stirrer at a moderate speed. The stained parasites are then centrifuged and washed twice with citrate saline and the pellet is resuspended in citrate saline in such a way so as to obtain the Aqueous Antigen Suspension having 48-50 million promastigotes per ml

### Preparation of The Composition

1 ml aliquots of Aqueous antigen with a promastigote count of 48 to 50 million, are taken in sterile glass injection vials or in sterile screw cap glass vials. To the above aliquots is added 100 ul of 0.3% sterile solution of sodium lauryl sulphate in citrate saline buffer. The contents are mixed homogeneously, the resulting solution is frozen at -30° C and freeze dried or lyophilised at -50° C to get freeze dried or lyophilised antigen composition as a brilliant blue coloured solid cake. The resulting freeze dried composition is sealed under vacuum or stored aseptically in air tight containers at ambient temperature. All the experimental operations are carried out with sterile medium and sterile reagent solutions under aseptic conditions.

### Diagnosis of Visceral Leishmaniasis

Test serum samples were serially diluted double fold (1:200 to 1:1,28,000) with 0.2% gelatin and 0.78% B-mercaptoethanol in physiological saline and 50 ul of these dilutions were placed in V-shaped well microtitre plates leaving the first well of the plate as antigen control containing 50 ul of only gelatin saline solution.

The Freeze Dried composition equivalent to 2.4 to 2.5 million promastigotes is taken in 50 ul of citrate saline buffer containing 0.25 ul of 1% solution of formaldehyde in citrate saline and added to 50 ul of test serum samples placed in V-shaped well microtitre plates. The contents are mixed well and kept at 22° C for 12 hrs and the results are read visually against a white back ground. A titre of 1:3200 or more was considered positive. The results are compared with control pool positive and pool negative sera. The test serum samples from patients suffering from visceral leishmaniasis show agglutination while serum samples from patients sufferring from tuberculosis, leprosy, amoebiasis, giardiasis, malaria, filariasis and trypanosomiasis did not show any sign of agglutination. The diagnostic test can be carried out by any one with ordinary skill in the art.

### Example 2:

### Preparation of AQ Antigen or Liquid Antigen

In-vitro, Leishmania donovani promastigotes were primarily grown by culturing infected hamster's spleen tissue in biphasic NNN medium at 26° C ± 1° C for 10 to 15 days. At every 7-8 days, Serial sub cultures of promastigotes are done by inoculating the biphasic NNN medium, consisting of 2 ml of NNN Medium and 2 ml of RPMI 1640 Overlay Medium, with 1x10⁶ to 1.5x10⁶ million promastigotes. Bulk cultivation of the promastigotes is done by inoculating 3 ml of NNN/RPMI 1640 sub culture containing 5x10⁶ promastigotes per ml in 50 ml of L-15 monophasic medium containing 10% Foetal Calf Serum. Parasites are harvested when a vast majority of promastigote population attains elongated form and their concentration reaches about 40x10⁶ promastigote per ml of the medium. The culture is centrifuged for 15 minutes at 4000 g at 4° C. The pellet formed after centrifugation is washed five times by centrifugation with 200 equal volumes of cold Locke's solution at 3200 g at 4° C for 10 minutes. One packed volume of promastigotes is treated with 20 equal volumes of 0.2% trypsin in Locke's solution (pH 7.2) for 45 minutes at 37°C and then centrifuged at 3200 g at 4° C and washed five times as above. The pellet is then suspended in cold Locke's solution so as to obtain a concentration of 200 x 10⁶ promastigotes per ml. An equal volume of cold 2% formaldehyde in Locke's solution is added and left at 4° C for 20 hours for fixation. The promastigotes are then centrifuged at 3200 g and washed with cold citrate saline. These promastigotes are stained with 0.1% (w/v) coomassie brilliant blue in citrate saline for 90 minutes using a magnetic stirrer at a moderate speed. The stained parasites are then centrifuged and washed twice with citrate saline and the pellet is resuspended in citrate saline in such a way so as to obtain the Aqueous Antigen Suspension having 48-50 million promastigotes per ml.

### Preparation of Composition

1 ml aliquots of Aqueous Antigen with a promastigote count of 48 to 50 million are taken in sterile glass injection vials or sterile screw cap glass vials. To the above aliquots is added 100 ul of 0.3% sterile solution of Sodium dodecyl sulphate in citrate saline buffer. The contents are mixed homogeneously, the resulting solution is frozen at -30° C and freeze dried or lyophilised at -50° C to get freeze dried or lyophilised antigen composition as a brilliant blue coloured solid cake. The freeze dried composition is sealed under vacuum or stored aseptically in air tight containers at ambient temperature. All the experimental operations are carried out with sterile medium and sterile reagent solutions under aseptic conditions.

### Diagnosis of Visceral Leishmaniasis

Test serum samples were serially diluted double fold (1:200 to 1:1,28,000) with 0.2% gelatin and 0.78% B-mercaptoethanol in physiological saline and 50 ul of these dilutions were placed in V-shaped well microtitre plates leaving the first well of the plate as antigen control containing 50 ul of only gelatin saline solution.

The Freeze Dried composition equivalent to 2.4 to 2.5 million promastigotes is taken in 50 ul of citrate saline buffer containing 0.25 ul of 1% solution of formaldehyde in citrate saline and added to 50 ul of test serum samples placed in V-shaped well microtitre plates. The contents are mixed well and kept at 22 °C for 12 hrs. and the results are read visually against a white back ground. A titre of 1:3200 or more was considered positive. The results are compared with control pool positive and pool negative sera. The test serum samples from patients suffering from visceral leishmaniasis show agglutination while serum samples from patients sufferring from tuberculosis, leprosy, amoebiasis, giardiasis, malaria, filariasis and trypanosomiasis did not show any sign of agglutination. The diagnostic test can be carried out by any one with ordinary skill in the art.

### Example 3:

### Preparation of AQ Antigen or Liquid Antigen

In-vitro, Leishmania donovani promastigotes were primarily grown by culturing infected hamster's spleen tissue in biphasic NNN medium at 26 °C ± 1 °C for 10 to 15 days. At every 7-8 days, serial sub cultures of promastigotes are done by inoculating the biphasic NNN medium, consisting of 2 ml of NNN Medium and 2 ml of RPMI 1640 Overlay Medium, with 1x10⁶ to 1.5x10⁶ million promastigotes. Bulk cultivation of the promastigotes is done by inoculating 3 ml of NNN/RPMI 1640 sub culture containing 5x10⁶ promastigotes per ml in 50 ml of L-15 monophasic medium containing 10% Foetal Calf Serum. Parasites are harvested when a vast majority of promastigote population attains elongated form and their concentration reaches about 40x10⁶ promastigote per ml of the medium. The culture is centrifuged for 15 minutes at 4000 g at 4° C. The pellet formed after centrifugation is washed five times by centrifugation with 200 equal volumes of cold Locke's solution at 3200 g at 4° C for 10 minutes. One packed volume of promastigotes is treated with 20 equal volumes of 0.2% trypsin in Locke's solution (pH 7.2) for 45 minutes at 37° C and then centrifuged at 3200 g at 4° C and washed five times as above. The pellet is then suspended in cold Locke's solution so as to obtain a concentration of 200 x 10⁶ promastigotes per ml. An equal volume of cold 2% formaldehyde in Locke's solution is added and left at 4° C for 20 hours for fixation. The promastigotes are then centrifuged at 3200 g and washed with cold citrate saline. These promastigotes are stained with 0.1% (w/v) coomassie brilliant blue in citrate saline for 90 minutes using a magnetic stirrer at a moderate speed. The stained parasites are then centrifuged and washed twice with citrate saline and the pellet is resuspended in citrate saline in such a way so as to obtain the Aqueous Antigen Suspension having 48-50 million promastigotes per ml.

### Preparation of Composition

1 ml aliquots of Aqueous Antigen with a promastigote count of 48 to 50 million, are taken in sterile glass injection vials or sterile screw cap glass vials. To the above aliquots is added 100 ul of 0.3% sterile sucrose solution in citrate saline buffer. The contents are mixed homogeneously, the resulting solution is frozen at -30° C and freeze dried or lyophilised at -50° C to get freeze dried or lyophilised antigen composition as a brilliant blue coloured solid cake. The resulting freeze dried product is sealed under vacuum or stored aseptically in air tight containers at ambient temperature. All the experimental operations are carried out with sterile medium and sterile reagent solutions under aseptic conditions.

### Diagnosis of Visceral Leishmaniasis

Test serum samples were serially diluted double fold (1:200 to 1:1,28,000) with 0.2% gelatin and 0.78% B-mercaptoethanol in physiological saline and 50 ul of these dilutions were placed in V-shaped well microtitre plates leaving the first well of the plate as antigen control containing 50 ul of only gelatin saline solution.

The Freeze Dried composition equivalent to 2.4 to 2.5 million promastigotes is taken in 50 ul of citrate saline buffer containing 0.25 ul of 1% solution of formaldehyde in citrate saline and added to 50 ul of test serum samples placed in V-shaped well microtitre plates. The contents are mixed well and kept at 22° C for 12 hrs and the results are read visually against a white back ground. A titre of 1:3200 or more was considered positive. The results are compared with control pool positive and pool negative sera. The test serum samples from patients suffering from visceral leishmaniasis show agglutination while serum samples from patients sufferring from tuberculosis, leprosy, amoebiasis, giardiasis, malaria, filariasis and trypanosomiasis did not show any sign of agglutination. The diagnostic test can be carried out by any one with ordinary skill in the art.

## Claims

1. A solid composition useful for the early diagnosis of visceral leishmaniasis obtained by freeze-drying a composition comprising trypsinised and Coomassie Briliant Blue stained Leishmania promastigotes and a protein stabilising solute, and containing from 0.002 mg to 1.0 mg protein stabilising solute per 100 million promastigotes,
wherein the protein stabilising solute is sodium lauryl sulphate, sodium dodecyl sulphate or sucrose.

2. A composition as claimed in claim 1, wherein the protein stabilising solute is sodium lauryl sulphate.

3. A composition as claimed in claim 1 or 2, wherein the composition is a powder.

4. A process for the preparation of the composition as claimed in any of claims 1 to 3, which comprises:
(a) preparing an aqueous antigen from Leishmania promastigotes by methods known per se;
(b) treating the aqueous antigen with a solution of protein stabilising solute in a buffer, wherein the protein stabilising solute is sodium lauryl sulphate, sodium dodecyl sulphate or sucrose;
(c) freezing the resulting solution;
(d) freeze drying or lyophilising the resulting frozen solution, and
(e) storing the freeze dried or lyophilised composition in sterile containers at ambient temperature.

5. A process as claimed in claim 4, wherein the Leishmania promastigotes are selected from *Leishmania donovani, Leishmania infantum and Leishmania chagasi.*

6. A process as claimed in claim 4, wherein the solution of the protein stabilising solute is prepared in water or physiological saline or citrate saline.

7. A process as claimed in claim 4, wherein the buffers used are selected from physiological saline and citrate saline.

8. A process as claimed in claim 4, wherein freezing of the solution in step (c) is performed between 0°C to -196°C.

9. A process as claimed in claim 4, wherein the freeze drying temperature of the frozen solution containing the aqueous antigen and protein stabilising solute ranges between 0°C to 100°C.

10. A process as claimed in claim 4, wherein the ambient temperature employed in step (c) is in the range of 18°C to 45°C.

11. A process as claimed in claim 4, wherein one ml of aqueous antigen contains Leishmania promastigotes in the range of 0.5 million to 100 million.

12. A method for the early diagnosis of visceral leishmaniasis by direct agglutination test, using the composition as claimed in any of claims 1 to 3, the said method comprising:
(a) taking the freeze dried composition in amounts equivalent to 0.5 million to 100 million promastigotes in 10 to 100 ul of citrate saline buffer containing 0.1 ul to 100 µl of 0.01% to 1% formalin solution in citrate saline;
(b) taking test dilution of pool positive, pool negative, blank and test serum samples separately in V shaped well microtitre plates in volumes ranging between 10µl to 100µl;
(c) mixing the suspension of the freeze dried composition of (a) to the pool positive, pool negative, blank and test serum samples of (b);
(d) keeping the contents (c) at 18°C to 40°C for a period of 2 to 20 hrs; and
(e) reading the results by visualising the agglutination in the well or formation of button at the bottom of the microtitre plates, wherein the agglutination is indicative of the positive reaction and the formation of the button is indicative of the negative reaction, the said positive reaction indicating Leishmania infectivity and the said negative reaction indicating no Leishmania infectivity.

## Patentansprüche

1. Feste Zusammensetzung, die zur Früherkennung von viszeraler Leishmaniose nützlich ist, welche durch Gefriertrockung einer Zusammensetzung erhalten wird, die trypsinierte und Coomassie-Brilliantblau-gefärbte Leishmaniose-Promastigoten und einen proteinstabilisierenden gelösten Stoff umfasst und 0,002 mg bis 1,0 mg proteinstabilisierenden gelösten Stoff pro 100 Millionen Promastigoten enthält,
wobei der proteinstabilisierende gelöste Stoff Natriumlaurylsulfat, Natriumdodecylsulfat oder Sucrose ist.

2. Zusammensetzung, wie in Anspruch 1 beansprucht, wobei der proteinstabilisierende gelöste Stoff Natriumlaurylsulfat ist.

3. Zusammensetzung, wie in Anspruch 1 oder 2 beansprucht, wobei die Zusammensetzung ein Pulver ist.

4. Verfahren zur Herstellung der Zusammensetzung, wie in einem der Ansprüche 1 bis 3 beansprucht, welches Folgendes umfasst:
(a) das Herstellen eines wässrigen Antigens aus Leishmaniose-Promastigoten durch an sich bekannte Verfahren;
(b) das Behandeln des wässrigen Antigens mit einer Lösung eines proteinstabilisierenden gelösten Stoffs in einem Puffer, wobei der proteinstabilisierende gelöste Stoff Natriumlaurylsulfat, Natriumdodecylsulfat oder Sucrose ist;
(c) das Einfrieren der resultierenden Lösung;
(d) das Gefriertrocknen oder Lyophilisieren der resultierenden gefrorenen Lösung, und
(e) das Lagern der gefriergetrockneten oder lyophilisierten Zusammensetzung in sterilen Behältern bei Umgebungstemperatur.

5. Verfahren, wie in Anspruch 4 beansprucht, wobei die Leishmaniose-Promastigoten aus *Leishmania donovani, Leishmania infantum* und *Leishmania chagasi* ausgewählt sind.

6. Verfahren, wie in Anspruch 4 beansprucht, wobei die Lösung des proteinstabilisierenden gelösten Stoffs in Wasser oder physiologischer Kochsalzlösung oder Citratsalzlösung hergestellt wird.

7. Verfahren, wie in Anspruch 4 beansprucht, wobei die verwendeten Puffer aus physiologischer Kochsalzlösung und Citratsalzlösung ausgewählt sind.

8. Verfahren, wie in Anspruch 4 beansprucht, wobei das Einfrieren der Lösung in Schritt (c) zwischen 0°C und -196°C durchgeführt wird.

9. Verfahren, wie in Anspruch 4 beansprucht, wobei die Gefriertrocknungstemperatur der gefrorenen, das wässrige Antigen und den proteinstabilisierenden gelösten Stoff enthaltenden Lösung im Bereich zwischen 0°C und -100°C liegt.

10. Verfahren, wie in Anspruch 4 beansprucht, wobei die in Schritt (e) eingesetzte Umgebungstemperatur im Bereich von 18°C bis 45°C liegt.

11. Verfahren, wie in Anspruch 4 beansprucht, wobei ein ml wässriges Antigen Leishmaniose-Promastigoten im Bereich von 0,5 Millionen bis 100 Millionen enthält.

12. Verfahren zur Früherkennung von viszeraler Leishmaniose durch einen direkten Agglutinationstest unter Verwendung der Zusammensetzung, wie in einem der Ansprüche 1 bis 3 beansprucht, wobei das Verfahren Folgendes umfasst:
(a) das Einsetzen der gefriergetrockneten Zusammensetzung in Mengen, die 0,5 Millionen bis 100 Millionen Promastigoten in 10 bis 100 µl Citratsalzlösungspuffer, enthaltend 0,1 µl bis 100 µl 0,01 %ige bis 1%ige Formalinlösung in Citratsalzlösung, entsprechen;
(b) das getrennte Einsetzen einer Testverdünnung von poolpositiven, poolnegativen Vergleichs- und Testserumproben in Mikrotiterplatten mit V-förmigen Mulden in Volumen im Bereich zwischen 10 µl und 100 µl;
(c) das Einmischen der Suspension der gefriergetrockneten Zusammensetzung von (a) in die poolpositiven, poolnegativen Vergleichs- und Testserumproben von (b);
(d) das Halten der Inhalte (c) bei 18°C bis 40°C für einen Zeitraum von 2 bis 20 Stunden; und
(e) das Ablesen der Resultate durch Sichtbarmachung der Agglutination in der Mulde oder Bildung eines Knopfs am Boden der Mikrotiterplatten, wobei die Agglutination die positive Reaktion anzeigt und die Bildung des Knopfs die negative Reaktion anzeigt, wobei die positive Reaktion auf eine Leishmaniose-Infektiosität hinweist und die negative Reaktion auf keine Leishmaniose-Infektiosität hinweist.

## Revendications

1. Composition solide utile pour le diagnostic précoce de la leishmaniose viscérale obtenue par séchage par congélation d'une composition comprenant des promastigotes de *Leishmania* trypsinisés et colorés au bleu de Coomassie brillant et un soluté de stabilisation des protéines, et contenant de 0,002 mg à 1,0 mg de soluté de stabilisation des protéines pour 100 millions de promastigotes,
dans laquelle le soluté de stabilisation des protéines est le sodium lauryl sulfate, le sodium dodécyl sulfate ou le sucrose.

2. Composition selon la revendication 1, dans laquelle le soluté de stabilisation des protéines est le sodium lauryl sulfate.

3. Composition selon la revendication 1 ou 2, dans laquelle la composition est une poudre.

4. Processus de préparation d'une composition selon l'une quelconque des revendications 1 à 3, qui comprend :
(a) la préparation d'un antigène aqueux de promastigotes de *Leishmania* par des procédés connus en soi ;
(b) le traitement de l'antigène aqueux avec une solution de soluté de stabilisation des protéines dans un tampon, dans lequel le soluté de stabilisation des protéines est le sodium lauryl sulfate, le sodium dodécyl sulfate ou le sucrose ;
(c) la congélation de la solution résultante ;
(d) le séchage par congélation ou la lyophilisation de la solution congelée résultante, et
(e) le stockage de la composition séchée par congélation ou lyophilisée dans des conteneurs stériles à température ambiante.

5. Processus selon la revendication 4, dans lequel les promastigotes de *Leishmania* sont choisis parmi *Leishmania donovani, Leishmania infantum* et *Leishmania chagasi.*

6. Processus selon la revendication 4, dans lequel la solution du soluté de stabilisation des protéines est préparée dans l'eau ou une solution saline physiologique ou une solution saline de citrate.

7. Processus selon la revendication 4, dans lequel les tampons sont choisis parmi une solution saline physiologique et une solution saline de citrate.

8. Processus selon la revendication 4, dans lequel la congélation de la solution à l'étape (c) est réalisée entre 0°C et -196°C.

9. Processus selon la revendication 4, dans lequel la température de séchage par congélation de la solution congelée contenant l'antigène aqueux et le soluté de stabilisation des protéines se trouve dans la plage de 0°C à -100°C.

10. Processus selon la revendication 4, dans lequel la température ambiante utilisée dans l'étape (c) se trouve dans la plage de 18°C à 45°C.

11. Processus selon la revendication 4, dans lequel 1 mL d'antigène aqueux contient des promastigotes de *Leishmania* dans la plage de 0,5 million à 100 millions.

12. Procédé de diagnostic précoce de la leishmaniose viscérale par un test d'agglutination direct, utilisant la composition selon l'une quelconque des revendications 1 à 3, ledit procédé comprenant :
(a) le placement de la composition séchée par congélation en des quantités équivalentes à 0,5 million à 100 millions de promastigotes dans 10 à 100 µL de tampon salin de citrate contenant de 0,1 µL à 100 µL de solution de formaline à 0,01 % à 1 % dans une solution saline de citrate ;
(b) le placement d'une dilution de test d'échantillons de sérum d'un pool positif, d'un pool négatif, de contrôle et de test séparément dans des plaques de microtitration à puits en forme de V en des volumes se trouvant dans la plage de 10 µL à 100 µL ;
(c) le mélange de la suspension de la composition séchée par congélation de (a) aux échantillons de sérum d'un pool positif, d'un pool négatif, de contrôle et de test de (b) ;
(d) la conservation des contenus (c) à 18°C à 40°C pendant une période de 2 à 20 heures ; et
(e) la lecture des résultats par visualisation de l'agglutination dans le puits ou la formation d'un bouton au fond des plaques de microtitration, dans lequel l'agglutination est un indicateur de la réaction positive et la formation du bouton est un indicateur de la réaction négative, ladite réaction positive indiquant une infection par *Leishmania* et ladite réaction négative n'indiquant pas d'infection par *Leishmania.*
